# DEMANDE DE BREVET EUROPEEN

(11) **EP 2 963 004 A1**
(43) Date de publication de la demande: **06.01.2016**
(21) Numéro de dépôt: 15305860.7
(22) Date de dépôt: 05.06.2015
(51) Int. Cl.: C07C 2/30

(54) **PROCÉDÉ AMELIORE DE DIMERISATION SELECTIVE DE L'ETHYLENE EN BUTENE-1**

(30) Priorité: 04.07.2014 FR 1456470
(71) Demandeur: IFP Énergies nouvelles, 92852 Rueil-Malmaison Cedex (FR)
(72) Inventeur: MAGNA, Lionel, 69007 LYON (FR); OLIVIER-BOURBIGOU, Helene, 69230 SAINT GENIS-LAVAL (FR)

(57) **Abrégé**

L'invention concerne un procédé de dimérisation sélective de l'éthylène en butène-1 mettant en oeuvre une composition catalytique comprenant au moins un composé alcoxy ou aryloxy du titane, au moins un additif choisi parmi les composés de type éther et au moins un composé d'aluminium.

## Description

La présente invention concerne la dimérisation sélective de l'éthylène en butène-1. Un objet de l'invention est de fournir un procédé de dimérisation de l'éthylène utilisant une composition catalytique particulière.

### Art Antérieur

Parmi les systèmes catalytiques capables de dimériser sélectivement l'éthylène en butène-1, il est possible d'identifier dans la littérature des systèmes catalytiques à base de vanadium (S. Zhang et al. Organometallics 2009, 28, 5925; K. Nomura et al. Inorg. Chem. 2013, 52, 2607), de fer ou de cobalt (S. Song et al. J. Organomet. Chem., 2011, 696, 2594; V. Appukuttan et al. Organometallics 2011, 30, 2285), de tungstène (H. Olivier et al. J. Mol. Catal. A: Chem. 1999, 148, 43; R. Tooze et al. Sasol Technology WO2005089940A2, 2005), de tantale (S. McLain et al. J. Am. Chem. Soc., 1978, 100(4), 1315; R. Schrock et al. Pure & App. Chem., 1980, 52, 729), de nickel (S. Mukherjee et al. Organometallics 2009, 28, 3074; K. Wang et al. Catal. Commun. 2009, 10, 1730; H. Liu et al. Dalton Trans. 2011, 40, 2614; J. Flapper et al. Organometallics 2009, 28, 3272, K. Song et al. Eur. J. Inorg. Chem. 2009, 3016) ou encore de Ti (A. W. Al-Sa'doun, Applied Catalysis A: General, 1993, 105, 1-40).

Parmi ces systèmes, ceux à base de titane occupent de loin une position privilégiée. Dans le brevet U.S. 2.943.125, K. Ziegler a décrit une méthode de dimérisation de l'éthylène en butène-1 au moyen d'un catalyseur obtenu par le mélange de trialkylaluminium et d'un tetraalcoolate de titane ou de zirconium. Lors de la réaction se forme également une certaine quantité de polyéthylène de haute masse moléculaire qui gêne considérablement la mise en oeuvre du procédé.

Plusieurs améliorations ont été proposées pour diminuer le taux du polyéthylène, en particulier dans le brevet U.S. 3.686.350 qui préconise l'emploi de composés organiques du phosphore conjointement avec les éléments du catalyseur, dans le brevet U.S. 4.101.600 qui décrit le traitement du catalyseur par de l'hydrogène ou dans le brevet U.S. 3.879.485 qui décrit l'utilisation de divers éthers comme solvants du milieu réactionnel. Bien que ces modifications du système catalytique initial apportent une amélioration à la sélectivité de la réaction, elles se révèlent d'une utilisation peu pratique, en particulier dans un procédé industriel dans lequel il faut pouvoir séparer le butène-1 du solvant en laissant seulement des traces de composé polaire dans les butènes.

De ce point de vue, le brevet FR 2 552 079 de la demanderesse, a démontré que la mise en oeuvre d'un catalyseur obtenu par l'interaction d'un trialkylaluminium d'une part, avec d'autre part un mélange préformé de titanate d'alkyl et d'additif de type éther en quantité stoechiométrique, améliore appréciablement l'activité et la sélectivité de ces catalyseurs pour la dimérisation de l'éthylène en butène-1. Le brevet FR 2 552 079 enseigne également que l'utilisation desdits additifs de type éther dans des rapports molaires supérieurs à 10 par rapport au titanate d'alkyle ralentit considérablement la réaction et aboutit à une sélectivité moins bonne.

S'il est connu que l'augmentation du rapport molaire entre l'alkylaluminium et le titanate d'alkyle conduit à une amélioration de la productivité, cela se fait dans les conditions du brevet FR 2 552 079, au détriment de l'opérabilité du procédé puisque des quantités de plus en plus importantes de polymère sont observées.

L'augmentation de la température de la réaction conduit également aux mêmes effets avec notamment une diminution de la stabilité du catalyseur et une augmentation de la proportion de polymère.

Le principal inconvénient des systèmes catalytiques à base de titane utilisés pour la formation sélective de butène-1 est donc la formation en quantité non négligeable de polymères. Cette formation de polymères peut être à l'origine d'une désactivation rapide du catalyseur et d'une difficulté accrue d'opérabilité.

Un objectif de l'invention est de fournir un procédé de dimérisation sélective de l'éthylène en butène-1 avec une production de polyéthylène réduite, voire quasi nulle et une opérabilité largement améliorée.

L'invention concerne un procédé de dimérisation sélective de l'éthylène en butène-1 mettant en oeuvre une composition catalytique comprenant au moins un composé alcoxy ou aryloxy du titane, au moins un additif choisi parmi les composés de type éther et au moins un composé d'aluminium, dans laquelle le rapport molaire entre l'additif et le composé de titane est strictement supérieur à 10 et le rapport molaire entre le composé d'aluminium et le composé alcoxy ou aryloxy du titane est strictement supérieur à 4.

Il a maintenant été trouvé, qu'un procédé mettant en oeuvre une composition catalytique comprenant au moins un composé alcoxy ou aryloxy du titane, au moins un additif choisi parmi les composés de type éther et au moins un composé d'aluminium, dans laquelle le rapport molaire entre l'additif et le composé alcoxy ou aryloxy du titane est strictement supérieur à 10 et le rapport molaire entre le composé d'aluminium et le composé alcoxy ou aryloxy du titane est strictement supérieur à 4, permettait d'obtenir une sélectivité très élevée pour la dimérisation sélective de l'éthylène en butène-1 et ceci avec une production de polyéthylène réduite, voire nulle.

### Description détaillée de l'invention

Dans la suite du texte et dans ce qui précède, le rapport molaire entre l'additif et le composé de titane sera, sauf indication contraire, exprimé en mole d'additif par mole de titane. Dans la suite du texte et dans ce qui précède, le rapport molaire entre le composé d'aluminium et le composé alcoxy ou aryloxy du titane sera, sauf indication contraire, exprimé en mole d'aluminium par mole de titane.

L'invention concerne un procédé de dimérisation sélective de l'éthylène en butène-1 mettant en oeuvre une composition catalytique comprenant au moins un composé alcoxy ou aryloxy du titane, au moins un additif choisi parmi les composés de type éther et au moins un composé d'aluminium, dans laquelle le rapport molaire entre ledit additif et le composé de titane est strictement supérieur à 10 et le rapport molaire entre le composé d'aluminium et le composé alcoxy ou aryloxy du titane est strictement supérieur à 4.

Avantageusement, le rapport molaire entre l'additif et le composé alcoxy ou aryloxy du titane de la composition catalytique est compris entre 11 et 19.

Avantageusement, le rapport molaire entre le composé d'aluminium et le composé alcoxy ou aryloxy du titane de la composition catalytique est compris entre 5 et 15.

Le composé alcoxy du titane utilisé dans la présente invention répond avantageusement à la formule générale [Ti(OR)₄] dans laquelle R est un radical alkyle linéaire ou ramifié comportant de 2 à 30 atomes de carbone. Le radical R peut comporter des substituants à base d'hétéroatome azoté, phosphoré, soufré et oxygéné.

Parmi les radicaux alcoxy préférés, on peut citer à titre d'exemple non limitatifs : le tétraéthoxy, tétraisopropoxy, le tétra-n-butoxy, le tétra-2-éthyl-hexyloxy.

Le composé aryloxy du titane utilisé dans la présente invention répond avantageusement à la formule générale [Ti(OR')₄] dans laquelle R' est un radical aryle substitué ou non par des groupements alkyle, aryle ou aralkyle comportant de 2 à 30 atomes de carbone. Le radical R' peut comporter des substituants à base d'hétéroatome azoté, phosphoré, soufré et oxygéné.

Parmi les radicaux aryloxy préférés, on peut citer à titre d'exemple non limitatif : le phénoxy, le 2-méthylphénoxy, le 2,6-diméthylphénoxy, , le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Le composé d'aluminium selon l'invention est avantageusement choisi dans le groupe formé par les composés hydrocarbylaluminium, les composés tris(hydrocarbyl)aluminium, les composés chlorés ou bromés d'hydrocarbylaluminium et les aluminoxanes.

Les tris(hydrocarbyl)aluminiums et les composés chlorés ou bromés d'hydrocarbylaluminium sont représentés par la formule générale AlR"ₘY₃₋ₘ dans laquelle R" est un radical hydrocarbyle, de préférence alkyle comprenant de 1 à 6 atomes de carbone, Y est un atome de chlore ou de brome, de préférence un atome de chlore et m est un nombre de 1 à 3.

De préférence, le composé d'aluminium est choisi dans le groupe formé par le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (*i*-Bu₂AlCl), le triéthylaluminium (AlEt₃), le tripropylaluminium (Al(*n*-Pr)₃), le triisobutylaluminium (Al(*i*-Bu)₃). Le composé d'aluminium préféré est le triéthylaluminium (AlEt₃).

L'additif de la composition catalytique selon l'invention est avantageusement choisi dans le groupe formé par l'éther diéthylique, le diisopropyléther, le 2-méthoxy-2-méthylpropane, 2-methoxy-2-méthylbutane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther et le benzofurane, pris seuls ou en mélange.

Une composition particulière selon l'invention est une composition dans laquelle le composé du titane est le [Ti(OⁿBu)₄], l'additif est le THF et est dans un rapport molaire (mol/mol) par rapport au composé du titane (THF/Ti) strictement supérieur à 10, de préférence compris entre 11 et 19 et le composé d'aluminium est le triéthylaluminium dans un rapport molaire (mol/mol) par rapport au composé du titane (AlEt₃/Ti) strictement supérieur à 4, de préférence compris entre 5 et 15.

### Procédé de préparation de la composition catalytique utilisée dans le procédé selon l'invention

La composition catalytique selon l'invention; c'est à dire le composé alcoxy ou aryloxy du titane, l'additif de type éther et le composé d'aluminium; peut être utilisé en mélange avec un solvant choisi dans le groupe formé par les hydrocarbures aliphatiques et cycloaliphatiques tels que l'hexane, le cyclohexane, l'heptane, le butane ou l'isobutane, par un hydrocarbure insaturé comme une monooléfine ou une dioléfine comportant par exemple de 4 à 20 atomes de carbone, par un hydrocarbure aromatique tel que le benzène, le toluène, l'ortho-xylène, le mésitylène, l'éthylbenzène ou par un hydrocarbure chloré tel que le chlorobenzène ou le dichlorométhane, purs ou en mélange. On utilise avantageusement les hydrocarbures aliphatiques comme le cyclohexane ou le n-heptane et les hydrocarbures aromatiques comme le toluène et l'ortho-xylène.

Selon un mode de préparation de la composition catalytique selon l'invention, on ajoute le composé d'aluminium dans une solution contenant l'additif et le composé alcoxy ou aryloxy du titane présent dans un rapport molaire strictement supérieur à 10, de préférence dans un rapport compris entre 11 et 19, le rapport molaire du composé d'aluminium sur le composé de titane étant strictement supérieur à 4, de préférence compris entre 5 et 15.

La concentration du titane dans la solution catalytique est avantageusement comprise entre 1.10⁻⁹ à 1 mol/L, de préférence entre 1.10⁻⁶ et 0,5 mol/L.

La température à laquelle les composants de la composition catalytique sont mélangés est avantageusement comprise entre -40 et +250°C, de préférence entre 0 et +150°C, par exemple à une température voisine de l'ambiante (15 à 30 °C). Le mélange peut être effectué sous une atmosphère d'éthylène ou de gaz inerte.

### Réaction de dimérisation

Le procédé selon l'invention est un procédé de dimérisation sélective de l'éthylène en butène-1 mettant en oeuvre la composition catalytique décrite ci-dessus.

La réaction de dimérisation de l'éthylène est avantageusement mise en oeuvre sous une pression totale de 0,5 à 20 MPa, de préférence de 0,5 à 10 MPa, et à une température de 20 à 180°C, de préférence de 40 à 140°C.

Selon un mode de réalisation, la réaction de dimérisation est mise en oeuvre en discontinu. On introduit un volume choisi de la composition catalytique, constituée comme décrit ci-dessus, avantageusement en solution, dans un réacteur muni des dispositifs habituels d'agitation, de chauffage et de refroidissement, puis on pressurise par de l'éthylène, avantageusement à la pression désirée, et on ajuste la température, de préférence à la valeur souhaitée. Le réacteur de dimérisation est maintenu à pression constante par introduction d'éthylène jusqu'à ce que le volume total de liquide produit représente, par exemple, de 2 à 50 fois le volume de la solution catalytique primitivement introduit. On détruit alors le catalyseur par tout moyen habituel connu de l'homme du métier, puis on soutire et on sépare les produits de la réaction et le solvant.

Selon un autre mode de réalisation préféré, la réaction catalytique de dimérisation de l'éthylène est mise en oeuvre en continu. Dans une première variante, on injecte séparément, dans un réacteur maintenue sous pression constante d'éthylène, d'une part une solution contenant le composé de titane et l'additif, et d'autre part une solution contenant le composé d'aluminium de manière à produire la composition catalytique selon l'invention. Ledit réacteur est agité par les moyens mécaniques classiques connus de l'homme du métier ou par une recirculation extérieure. La température et la pression d'éthylène sont maintenues constantes aux valeurs souhaitées en utilisant les moyens classiques connus de l'homme du métier. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

Dans une seconde variante, on injecte dans un premier réacteur/mélangeur, d'une part une solution contenant le composé du titane et l'additif, et d'autre part le composé d'aluminium de manière à produire la composition catalytique selon l'invention, ladite composition est ensuite introduite en continu dans un réacteur maintenu sous pression constante d'éthylène. Ce mélange dans le premier réacteur/mélangeur peut être réalisé sous atmosphère inerte ou sous atmosphère d'éthylène. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

### Produits obtenus

Le procédé selon l'invention permet la production sélective de butène-1. Ce composé trouve une utilisation en tant que comonomères avec l'éthylène dans la fabrication du polyéthylène basse densité linéaire.

Les exemples suivants illustrent l'invention sans en limiter la portée.

### Exemples

### Exemples 1-4 :

Les essais de dimérisation de l'éthylène présentés dans le tableau 1 ci-dessous ont été réalisés dans un autoclave en acier inoxydable d'un volume utile de 500 mL, muni d'une double enveloppe permettant de réguler la température par circulation d'huile. L'agitation est assurée par une pale Rushton à entraînement mécanique.

Dans ce réacteur on introduit sous atmosphère d'éthylène et à température ambiante, 40 mL de n-heptane ainsi que 5 mL d'une solution à 0,085 mol/L du composé de titane dans le n-heptane. Une fois la température du réacteur portée à 53°C, on introduit sous pression d'éthylène la quantité de co-catalyseur à base d'aluminium souhaité (préalablement dilué dans 5 mL du n-heptane). La pression d'éthylène est maintenue à 23 MPa et la température à 53°C. Après 1 h de réaction, l'introduction d'éthylène est arrêtée et le réacteur refroidi à 25°C. Le réacteur est ensuite dégazé au travers d'un compteur à gaz. Ce gaz est analysé par chromatographie en phase gazeuse. La phase liquide contenue dans le réacteur est ensuite pesée et analysée par chromatographie en phase gazeuse. Le polymère produit est récupéré, séché et pesé.

La composition des produits obtenus est donnée dans le tableau 1 ci-après. Dans ce tableau, l'activité est définie comme la masse d'éthylène consommée par gramme de titane introduit initialement et par heure. La distribution C4 (%C₄) est la quantité d'oléfines ayant un nombre d'atomes de carbone égal à 4 dans la distribution totale. Le pourcentage %C₄ ⁼¹ représente la sélectivité en produit linéaire butène-1 dans la coupe C4. La quantité de polymère (%PE) correspond à la masse de polymère récupérée, ramenée dans la distribution totale.

**Tableau 1:**

| Ex. | Composé de Ti | Additif | Ratio molaire « Additif/Ti » | Co-catalyseur « Al » | Ratio molaire Al/Ti | T (°C) | t (min) | Activité (g/gTi/h) | % C₄ (%C₄⁼¹) | % PE |
|---|---|---|---|---|---|---|---|---|---|---|
| | | | | | | | | | | |
| 1 | [Ti(OⁿBu)₄] | THF | 4 | AlEt₃ | 3.0 | 53 | 87 | 6200 | 94 (99⁺) | 0.05 |
| 2 | [Ti(OⁿBu)₄] | THF | 4 | AlEt₃ | 6.8 | 53 | 62 | 12400 | 94 (99⁺) | 0.30 |
| 3 | [Ti(OⁿBu)₄] | THF | 10.7 | AlEt₃ | 6.8 | 53 | 114 | 6800 | 94 (99⁺) | nd* |
| 4 | [Ti(OⁿBu)₄] | THF | 18.8 | AlEt₃ | 6.9 | 53 | 125 | 7500 | 95 (99⁺) | nd* |

| | | | | | | | | | | |
|---|---|---|---|---|---|---|---|---|---|---|
| * nd= non détecté (cette dénomination caractérise l'absence totale de polymère) | | | | | | | | | | |

Dans ce tableau, les exemples 1 et 2 montrent que les compositions ayant un rapport molaire THF/Ti<10 (exemples comparatifs non conformes à l'invention), l'augmentation du rapport molaire AlEt₃/Ti de 3 à 6,8 conduit à une augmentation notable de la production de polyéthylène.

Les exemples 3 et 4 conformes à l'invention démontrent que les compositions ayant un rapport molaire THF/Ti strictement supérieur à 10 présentent sous un ratio AEt₃/Ti de 6.8 (cf. Ex.2) de très bonnes activité et sélectivité pour la dimérisation sélective de l'éthylène en butène-1 et ceci sans la production de polyéthylène.

## Revendications

1. Procédé de dimérisation sélective de l'éthylène en butène-1 mettant en oeuvre une composition catalytique comprenant au moins un composé alcoxy ou aryloxy du titane, au moins un additif choisi parmi les composés de type éther et au moins un composé d'aluminium, dans laquelle le rapport molaire entre l'additif et le composé alcoxy ou aryloxy du titane est strictement supérieur à 10 et le rapport molaire entre le composé d'aluminium et le composé alcoxy ou aryloxy du titane est strictement supérieur à 4.

2. Procédé selon la revendication 1 dans lequel le rapport molaire entre l'additif et le composé alcoxy ou aryloxy du titane de la composition catalytique est compris entre 11 et 19.

3. Procédé selon la revendication 1 ou 2 dans lequel le rapport molaire entre le composé d'aluminium et le composé alcoxy ou aryloxy du titane de la composition catalytique est compris entre 5 et 15.

4. Procédé selon l'une des revendications 1 à 3 dans lequel le composé alcoxy du titane répond à la formule générale [Ti(OR)₄] dans laquelle R est un radical alkyle linéaire ou ramifié comportant de 2 à 30 atomes de carbone.

5. Procédé selon l'une des revendications 1 à 3 dans lequel le composé aryloxy du titane répond à la formule générale [Ti(OR')₄] dans laquelle R' est un radical aryle substitué ou non par des groupements alkyle, aryle ou aralkyle comportant de 2 à 30 atomes de carbone.

6. Procédé selon l'une des revendications précédentes dans lequel le composé d'aluminium est choisi dans le groupe formé par les composés hydrocarbylaluminium, les composés tris(hydrocarbyl)aluminiums, les composés chlorés ou bromés d'hydrocarbylaluminium et les aluminoxanes.

7. Procédé selon la revendication 6 dans lequel le composé d'aluminium est choisi dans le groupe formé par le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (*i*-Bu₂AlCl), le triéthylaluminium (AlEt₃), le tripropylaluminium (Al(*n*-Pr)₃), le triisobutylaluminium (Al(*i*-Bu)₃).

8. Procédé selon l'une des revendications précédentes mettant en oeuvre une composition dans laquelle le composé du titane est le [Ti(OⁿBu)₄], l'additif est le THF et est dans un rapport molaire par rapport au composé du titane (THF/Ti) strictement supérieur à 10 et le composé d'aluminium est le triéthylaluminium dans un rapport molaire par rapport au composé du titane (AlEt₃/Ti) strictement supérieur à 4.

9. Procédé selon l'une des revendications précédentes dans lequel l'additif est choisi dans le groupe formé par l'éther diéthylique, le diisopropyléther, le 2-méthoxy-2-méthylpropane, 2-methoxy-2-méthylbutane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther et le benzofurane, pris seuls ou en mélange.

10. Procédé selon l'une des revendications précédentes dans lequel la composition catalytique est utilisé en mélange avec un solvant choisi dans le groupe formé par les hydrocarbures aliphatiques et cycloaliphatiques, par un hydrocarbure insaturé comme une monooléfine ou une dioléfine comportant par exemple de 4 à 20 atomes de carbone, par un hydrocarbure ou par un hydrocarbure chloré, purs ou en mélange.

11. Procédé selon l'une des revendications précédentes mis en oeuvre sous une pression totale de 0,5 à 20 MPa et à une température de 20 à 180°C.

12. Procédé selon l'une des revendications précédentes mis en oeuvre dans un mode de réalisation discontinu ou continu.

13. Procédé selon l'une des revendications précédentes dans lequel on introduit un volume choisi de la composition catalytique selon l'une des revendications 1 à 10 dans un réacteur muni de dispositifs d'agitation, de chauffage et de refroidissement, puis on pressurise par de l'éthylène et on ajuste la température.

14. Procédé de dimérisation sélective de l'éthylène en butène-1 selon l'une des revendications précédentes mis en oeuvre tel que on introduit séparément, dans un réacteur maintenu sous pression constante d'éthylène, d'une part une solution contenant le composé du titane et l'additif, et d'autre part une solution contenant le composé d'aluminium de manière à produire la composition catalytique telle définie selon l'une des revendications 1 à 10.

15. Procédé de dimérisation sélective de l'éthylène en butène-1 selon l'une des revendications précédentes mis en oeuvre tel que on introduit dans un premier réacteur/mélangeur, d'une part une solution contenant le composé du titane et l'additif, et d'autre part le composé d'aluminium de manière à produire la composition catalytique telle définie selon l'une des revendications 1 à 10, ladite composition étant ensuite introduite en continu dans un réacteur maintenu sous pression constante d'éthylène.

16. Procédé de préparation de la composition catalytique selon l'une des revendications 1 à 10 selon lequel on ajoute le composé d'aluminium dans une solution contenant l'additif et le composé alcoxy ou aryloxy du titane présent dans un rapport molaire strictement supérieur à 10, le rapport molaire du composé d'aluminium sur le composé de titane étant strictement supérieur à 4.
